# EUROPÄISCHE PATENTSCHRIFT

(11) **EP 0 750 191 B1**
(45) Veröffentlichungstag und Bekanntmachung des Hinweises auf die Patenterteilung: **24.09.2003**
(21) Anmeldenummer: 96112470.8
(22) Anmeldetag: 17.10.1990
(51) Int. Cl.: G01N 33/00

(54) **Gas-Sensor-Anordnung**
Gas sensor arrangement
Dispositif détecteur de gaz

(30) Priorität: 17.10.1989 DE 3934532; 23.06.1990 DE 4020113; 08.08.1990 DE 4025117; 31.08.1990 DE 4027547
(43) Veröffentlichungstag der Anmeldung: 27.12.1996
(62) Teilanmeldung aus: 90915426.2
(73) Patentinhaber: paragon AG, 33129 Delbrück (DE)
(72) Erfinder: Rump, Hanns, 59427 Unna-Massen (DE); Kohl, Claus-Dieter, 35396 Giessen (DE)
(74) Vertreter: Lelgemann, Karl-Heinz, Dipl.-Ing.

(56) Entgegenhaltungen:
- EP-A- 0 102 067
- WO-A-82/03689
- DE-A- 2 816 331
- DE-A- 3 213 286
- DE-A- 3 221 132
- DE-A- 3 836 819
- GB-A- 2 170 913
- JP-A- 61 093 945
- US-A- 3 748 625
- US-A- 3 961 248
- US-A- 4 572 900
- US-A- 4 733 605
- US-A- 4 744 289
- PATENT ABSTRACTS OF JAPAN vol. 8, no. 153 (P-287), 17.Juli 1984 & JP 59 050353 A (MATSUSHITA DENKO KK), 23.März 1984,
- W. M. SEARS: "THE GAS SENSING PROPERTIES OF SINTERED BISMUTH IRON MOLYBDATE CATALYST" SENSORS AND ACTUATORS, Bd. 19, Nr. 4, 15.September 1989, LAUSANNE, CH, Seiten 351-370, XP000048429

## Beschreibung

Die Erfindung bezieht sich auf eine Gas-Sensor-Anordnung nach dem Oberbegriff des Patentanspruchs 1.

Die Notwendigkeit, Gase in der Luft nachzuweisen, wird immer wichtiger.

Es besteht z.B. der Wunsch, in Abhängigkeit vom Schadstoffgehalt der Luft Schaltvorgänge auszulösen. Insbesondere bei der Belüftung von Kraftfahrzeugen sind zahlreiche Bemühungen bekannt, mit Hilfe eines gasempfindlichen Sensors so auf die Lüftung des Kraftfahrzeugs einzuwirken, daß bei Bestehen einer ungewöhnlich hohen Schadstoffbelastung der Außenluft die Frischluftzufuhr des Kraftfahrzeugs unterbrochen und z.B. auf Umluftbetrieb umgeschaltet wird.

Es ist bekannt, mit Hilfe von Metalloxid-Gas-Sensorelementen bestimmte Gase zu detektieren. Dabei besteht das Sensorelement meist aus einem Metalloxid, das durch eine geeignete Heizung auf eine Arbeitstemperatur gebracht wird.

Große Verbreitung haben Zinndioxid-Sensorelemente gefunden, die auf eine Temperatur von ca. 250 Grad C bis 450 Grad C aufgeheizt werden. Dem Zinndioxid wird in der Regel eine katalytisch wirkende Substanz beigemischt. Bewährt hat sich z.B. Platin, Palladium, Rhodium.

Sensorelemente dieser Art reagieren mit einer Widerstandsverminderung bei der Anwesenheit von Gasen, die oxidierbar sind. Dabei gibt das Zinndioxid Sauerstoff ab. Wird der Sensor wieder normaler Luft ausgesetzt, reagiert die Oberfläche mit dem Luftsauerstoff wieder zu Zinndioxid. Der Vorgang ist also reversibel und unterliegt keinem verschleiß.

Es wird beobachtet, daß mitunter eine grobe Differenz zwischen erwartetem Sensorleitwert und Konzentration oxidierbarer Gase, z.B. Kohlenmonoxid, auftritt. Nähere Untersuchungen haben jetzt gezeigt, daß bei gleichzeitiger Anwesenheit reduzierbarer Gase diese die Reaktion der oxidierbaren Gase mit der Sensoroberfläche stark beeinflussen. Im Extremfall wird trotz grosser Konzentration keine oder eine nur geringe elektrische Reaktion des Sensors erfolgen. Beispielhaft sei auf die Reaktion bei gleichzeitigem Vorhandensein von Kohlenmonoxid (CO) und Stickstoffoxid (NOx) hingewiesen. Ursache ist die direkte Reaktion der Gase miteinander in der Nähe der heißen Sensoroberfläche, wobei die Katalysatorsubstanz Einfluß nimmt.

Insbesondere ist bei dem vorstehend erwähnten beheizten Zinndioxid-Sensor nachteilig, daß er gegenüber Dieselabgasen wenig sensibel ist. Dies ergibt sich aus folgendem:

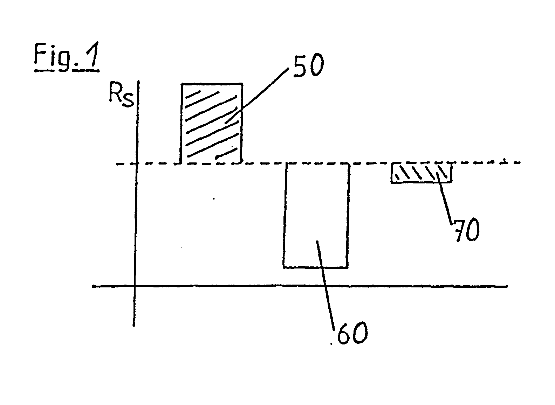

Zinndioxid-Sensoren reagieren mit einer Widerstandsverminderung immer dann, wenn eine oxidierbare, gasförmige Substanz anwesend ist. Zinndioxid-Sensoren reagieren also z.B. auf Kohlenmonoxid, Wasserstoff oder Benzindämpfe entsprechend der Darstellung des Balkens 60 in Figur 1. In den Abgasen von Benzinmotoren befinden sich diese Bestandteile, so daß es zu dieser deutlichen Reaktion des Zinndioxid-Sensors kommt.

Wird dieser Zinndioxid-Sensor im Labor mit Stickoxiden (NOx) beaufschlagt, erhöht er seinen inneren Widerstand, wie durch den Balken 50 in Figur 1 gezeigt.

Im Abgas eines Diesel-Motors, insbesondere wenn dieser unter Last betrieben wird, befinden sich neben den dem Balken 60 in Figur 1 entsprechenden Substanzen auch die dem Balken 50 entsprechenden Substanzen. Als Resultat wird beobachtet, daß der Zinndioxid-Sensor deutlich weniger reagiert, als man es aufgrund der meßtechnisch nachgewiesenen Gasanteile vermuten kann. Die Reaktion des Zinndioxid-Sensors entspricht dem Balken 70 in Figur 1.

Dies ist für den Betrieb des Sensors sehr störend, da beim Einsatz eines solchen Gas-Sensors in der Praxis häufig sowohl oxidierbare als auch reduzierbare Gase auftreten.

Aus der JP61-93945 ist es bekannt, den Widerstandswert eines NO-empfindlichen Zinndioxid sensor elements mit Fe-Beimengung zu verwenden, um in Abhängigkeit vom NO-Gehalt der Außenluft ein Schaltsignal für die Innenraumbelüflung eines Kfz zur VerFügung zu stellen.

Aus der DE-C-32 13 286 ist eine Gas-Sensor-Anordnung zur Detektion von Gasen in der Luft bekannt, bei der bereits mehrere Meßfühler bzw. Sensorelemente an einem Träger vorgesehen sind, wobei es sich hierbei um unterschiedliche Sensorelemente, die unterschiedliche Stoffe erfassen sollen, handelt. Der Betrieb der Gas- Sensor-Anordnung erfolgt bei einer Temperatur, die für sämtliche verwendeten Sensorelemente gleichmäßig ist und so ausgewählt ist, daß alle Sensorelemente bei dieser Temperatur mit befriedigenden Ergebnissen funktionieren.

Jeweils einem Meßfühler wird eine definierte und bestimmte Spannung zugeordnet, wobei diese Spannung dann als feste Größe in ein lineares Gleichungssystem eingeführt wird. Dieses lineare Gleichungssystem umfaßt entsprechend der Anzahl der Meßfühler sechs Gleichungen, wobei es zur Berechnung des Anteils von sechs unterschiedlichen Gasen im Gasvolumen dient. Die einzelnen Meßfühler haben unterschiedliche Empfindlichkeiten den zu erfassenden Gasen gegenüber.

Die US 3 961 248 zeigt eine Gas-Sensor-Anordnung, zu der mehrere Sensorelemente aus Zinndioxid mit underschiedlichen Beimengungen an Katalysator material gehören. Bei einigen Ausführungsformen sind zwei Sensorelemente vorhanden, die der Erfassung oxi-dierbarer Gase mit jeweils unterschiedlichen Empfindlichkeiten dienen. Im Ausführungsbeispiel der Figur 11 bilden die beiden Sensorelemente einen Spannungsteiler. Der Signalwert, der zwischen den beiden Sensorelementen abgegriffen wird, wird von beiden Sensorelementen bzw. von den Gasbeaufschlagungen der beiden Sensorelemente beeinflußt.

Ausgehend von dem eingangs geschilderten Stand der Technik liegt der Erfindung die Aufgabe zugrunde, eine Gas-Sensor-Anordnung zu schaffen, mit der es möglich sein soll, sowohl bei Vorhandensein von Ottomotorabgasen in der Außenluft als auch bei Vorhandensein von Dieselmotorabgesen in der Außenluft unmittelbar einen Schaltvorgang auszulösen.

Diese Aufgabe wird erfindungsgemäß durch die Merkmale im kennzeichnenden Teil des Patentanspruchs 1 gelöst.

Erfindungsgmeäß wird eine Gas-Sensor-Anordnung geschaffen, mittels der es möglich ist, immer dann ein Schaltsignal zu erzeugen, wenn eine beliebig vorgebbare und einstellbare Schadstoffkonzentration in der Umgebungsluft überschritten oder unterschritten wird. Hierbei ist es unerheblich, ob die Schadstoffkonzentration durch das Vorhandensein von Dieselabgasen, von Benzinabgasen oder von Diesel- und Benzinabgasen gebildet wird. In jedem Fall ist durch die erfindungsgemäße Gas-Sensor-Anordnung sichergestellt, daß bei jeder der genannten Schadstoffkonzentrationen ein Schaltsignal auslösbar ist.

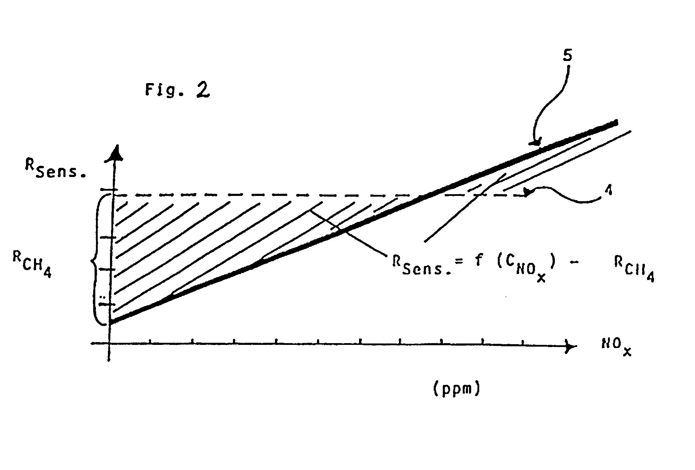

Figur 2 zeigt den prinzipiellen Zusammenhang, wobei der innere Sensorwiderstand 5 bei Anwesenheit einer konstanten Konzentration von Methan (CH4) und einer unterschiedlichen Konzentration von Stickstoffoxid (NO2) gezeigt wird. Als 100%-Widerstandswert 4 wird der Sensorwiderstand bei Normalluft bezeichnet.

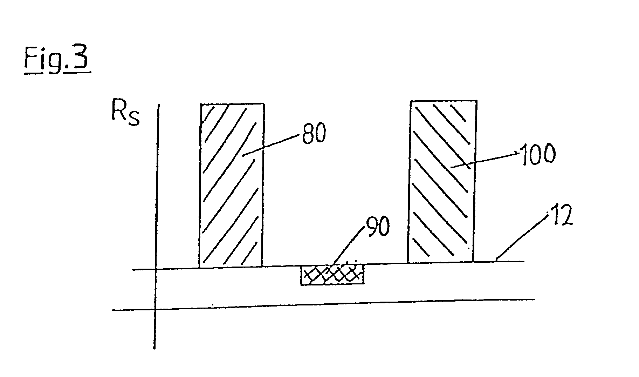

Meßergebnisse für ein zweites Sensorelement 21, die prinzipiell in Figur 3 dargestellt sind, wurden bei den nachstehend aufgeführten Werkstoffen erreicht.

Figur 3 zeigt
a) einen in bezug auf übliche Zinndioxid-Sensoren ungewöhnlich niedrigen Sensorwiderstand 12 des Sensorelements bei unbelasteter Luft;
b) eine deutliche Reaktion des Sensorelements bei der Beaufschlagung mit Stickoxiden, wie sich aus dem Balken 80 ergibt;
c) eine geringe Reaktion bei der Beaufschlagung mit Abgasen aus Otto-Motoren, wie sich aus dem Balken 90 ergibt;
d) eine deutliche Reaktion des Sensorelements bei der Beaufschlagung mit Dieselabgasen, wie sich aus dem Balken 100 ergibt.

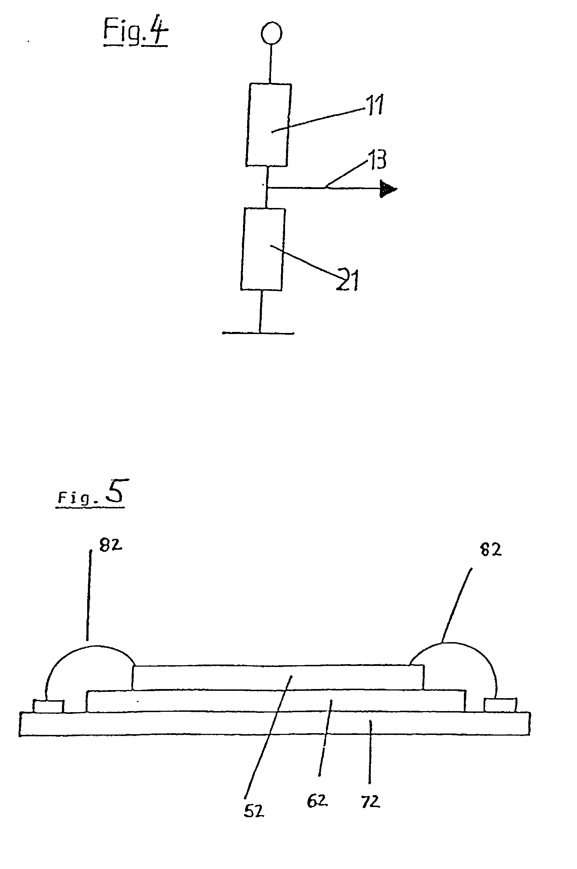

Hieraus ergibt sich, daß eine Gas-Sensor-Anordnung, die ein übliches erstes Zinndioxid-Sensorelement 11 in Kombination mit dem zweiten Sensorelement 21, dessen Funktionsweise in Figur 3 dargestellt ist, in einer Beschaltung aufweist, die der Figur 4 entspricht, für die Anwendung im Kraftfahrzeug sehr gute Ergebnisse bringt.

Figur 4 zeigt eine prinzipielle Darstellung, die sich die Tatsache zunutze macht, daß das vorstehend beschriebene zweite Sensorelement 21 bei unbelasteter Luft einen sehr niedrigen Innenwiderstand hat. Hierbei bilden das erste Zinndioxid-Sensorelement 11 und das zweite Sensorelement 21 einen Spannungsteiler. Dessen Abgriff 13 stellt ein elektrisches Signal zur Verfügung, das unabhängig vom eingangs beschriebenen Maskierungseffekt ein Schaltsignal zur Verfügung stellt, das sowohl bei Diesel- als auch bei Otto-Motoren brauchbare Werte ergibt.

Für das die Eigenschaften gemäß Figur 3 aufweisende zweite Sensorelement 21 wurden folgende Sensorwerkstoffe verwendet:
a) Zinndioxid oder reduziertes Zinndioxid mit Beimengungen von katalytischen Materialien. In der erprobten Ausführung wurden dem Zinndioxid zwischen 1 - 15 % Palladium beigemengt.
b) Wie a), jedoch wurde Palladium durch Platin ersetzt. Bei einer erprobten Ausführungsform wurden mit einer katalytischen Beimengung zwischen 1 - 10 % Platin Versuche gemacht.
c) Eisenoxid mit 1 - 20 % Palladium als katalytische Beimengung.
d) Ein Gemisch aus Zinndioxid, Zinkoxid und Palladium. Erprobt wurden ca. 50 % Zinndioxid, ca. 40 % Zinkoxid, 1 - 10 % Palladium.
e) Weiterhin wird vorgeschlagen, durch Aufdampfen oder Sputtern eine dünne Edelmetallauflage aus Gold oder Platin auf das Sensormaterial zu schaffen. Oxidierbare Substanzen reagieren unter Einfluß des Katalysators mit dem Luftsauerstoff und erreichen das Sensormaterial nicht.
f) Draht oder Drahtwendel, bevorzugt aus Katalysatoren, evtl. beheizt, sind in der Nähe der Sensoroberfläche angeordnet, um oxidierbare Stoffe mit Luftsauerstoff zu oxidieren. Der Zweck entspricht e).
g) Allen Sensorwerkstoffen ist gemeinsam, daß es sich um n-leitende Halbleiter handelt (im Gegensatz zu z.B. Phthalocyanin-Sensoren, die p-leitend sind).

Ein Zinndioxid-Sensorelement üblicher Funktionsweise wird mit Gas-Sensoren üblicher Konstruktion kombiniert, wobei die Sensorelemente letztgenannter Gas-Sensoren n-leitend sind und aus Zinndioxid mit katalytischen Beimengungen, Zinkoxid mit katalytischen Beimengungen, einer Mischung aus Zinndioxid und Zinndioxyd mit katalytischen Beimengungen und aus Eisenoxid mit katalytischen Beimengungen bestehen.

Als katalytische Beimengung wird Palladium, Platin oder Rhodium vorgeschlagen.

Die Erfindung ist überall dort vorteilhaft einsetzbar, wo Gase detektiert werden müssen und wo die Gefahr besteht, daß oxidierbare Gase mit sauerstofftragenden Gasen, z.B. Stickoxiden, gemeinsam auftreten. Hier ist insbesondere in der Umwelttechnik eine Anwendung vorteilhaft möglich.

In der bevorzugten Ausführungsform ergibt sich ein Aufbau nach Figur 5. Ein Träger 72 ist aus Aluminiumoxid oder Silizium. Auf ihm ist eine Heizung 62 aufgebracht, die vorzugsweise aus gewendeltem, in Dünn- oder Dickfilmtechnik aufgebrachtem Platin besteht. Davon isoliert ist das eigentliche Sensormaterial 52 aufgebracht, das z.B. ein Wismut-Molybdat ist. Über Kontaktierungen 82 wird der elektrische Leitwert des Molybdats meßbar gemacht.

## Patentansprüche

1. Gas-Sensor-Anordnung mit zumindest einem ersten Sensor-element (11), welches als Zinndioxid sensorelement ausgebildet ist und hauptsächlich empfindlich gegenüber oxidierbaren Gasen in Außenluft ist und ein dementsprechendes Meßsignal erzeugt, zumindest einem zweiten Sensorelement (21), und einer elektrischen Steuer- und Auswerteeinheit, in die die vom ersten und zweiten Sensorelement (11, 21) erzeugten Meßsignale eingegeben werden, **dadurch gekennzeichnet, daß** das zweite Sensorelement (21) hauptsächlich empfindlich gegenüber reduzierbaren Gasen in Außenluft ist und ein dementsprechendes Meßsignal erzeugt, und daß das Meßsignal des ersten Sensorelements (11) und das Meßsignal des zweiten Sensorelements (21) an einem den beiden Sensorelementen (11, 21) gemeinsamen Abgriff (13) zur Verfügung gestellt werden, wobei das erste Sensorelement (11) und das zweite Sensorelement (21) einen Spannungsteiler bilden, der den Abgriff (13) aufweist und wobei die Anordnung ein Schaltsignal sowohl bei Vorhaudensein von Dieselmotorabgasen als auch bei Vorhandensein von Ottomotorabgasen zur Verfügung stellt.

2. Gas-Sensor-Anordnung nach Anspruch 1, bei der das zweite Sensorelement (21) ein n-leitendes Gas-Sensorelement auf der Basis eines homogenen Halbleiters ist und in der gasempfindlichen Schicht aus Metalloxid besteht.

3. Gas-Sensor-Anordnung nach Anspruch 2, bei der das zweite Sensorelement (21) in der gasempfindlichen Schicht aus reduziertem Zinnoxid besteht.

4. Gas-Sensor-Anordnung nach Anspruch 2 oder 3, bei der das zweite Sensorelement (21) katalytische Beimengungen aufweist und auf die gasempfindliche Schicht eine dünne Edelmetallschicht aufgebracht ist.

5. Gas-Sensor-Anordnung nach einem der Ansprüche 2 bis 4, bei der das zweite Sensorelement (21) von einem beheizten Draht aus katalytischem Material, wie Platin, Gold, Rhodium oder Palladium umschlossen ist.

6. Gas-Sensor-Anordnung nach Anspruch 2, bei der das zweite Sensorelement (21) aus Zinndioxid mit katalytischen Beimengungen, Zinkoxid mit katalytischen Beimengungen oder Eisenoxid mit katalytischen Beimengungen besteht.

7. Gas-Sensor-Anordnung nach Anspruch 2, bei der das zweite Sensorelement (21) aus einer Mischung von mindestens zwei der im Anspruch 8 genannten Materialien besteht.

8. Gas-Sensor-Anordnung nach einem der Ansprüche 2 bis 7, bei der im zweiten Sensorelement (21) als katalytische Beimengungen Palladium, Platin, Rhodium oder Gold Verwendung finden.

9. Verfahren zur Detektion von Gasen, bei dem zur Erkennungssicherheit gegenüber Abgasen von Diesel-, Katalysator- oder herkömmlichen Otto-Motoren eine Gas-Sensor-Anordnung nach einem der Ansprüche 1 - 8 eingesetzt wird.

## Claims

1. Gas sensor arrangement having at least one first sensor element (11) which is in the form of a tin dioxide sensor element and is mainly sensitive to oxidisable gases in outside air and generates a corresponding measurement signal, at least one second sensor element (21), and an electrical control and evaluation unit into which the measurement signals generated by the first and second sensor element (11, 21) are entered, **characterised in that** the second sensor element (21) is mainly sensitive to reducible gases in outside air and generates a corresponding measurement signal, and **in that** the measurement signal of the first sensor element (11) and the measurement signal of the second sensor element (21) are made available at a tap (13) common to the two sensor elements (11, 21), the first sensor element (11) and the second sensor element (21) forming a voltage divider incorporating the tap (13), and the arrangement making a switching signal available both in the case of the presence of diesel engine exhaust gases and in the case of the presence of Otto engine exhaust gases.

2. Gas sensor arrangement according to claim 1, wherein the second sensor element (21) is an N-type gas sensor element based on a homogeneous semiconductor and comprises metal oxide in the gas-sensitive layer.

3. Gas sensor arrangement according to claim 2, wherein the second sensor element (21) comprises reduced tin oxide in the gas-sensitive layer.

4. Gas sensor arrangement according to claim 2 or 3, wherein the second sensor element (21) has catalytic additives, and a thin layer of noble metal is applied to the gas-sensitive layer.

5. Gas sensor arrangement according to any one of claims 2 to 4, wherein the second sensor element (21) is surrounded by a heated wire of catalytic material, such as platinum, gold, rhodium or palladium.

6. Gas sensor arrangement according to claim 2, wherein the second sensor element (21) comprises tin dioxide with catalytic additives, zinc oxide with catalytic additives or iron oxide with catalytic additives.

7. Gas sensor arrangement according to claim 2, wherein the second sensor element (21) comprises a mixture of at least two of the materials mentioned in claim 8.

8. Gas sensor arrangement according to any one of claims 2 to 7, wherein palladium, platinum, rhodium or gold is used in the second sensor element (21) as catalytic additive.

9. Method for detecting gases, wherein a gas sensor arrangement according to any one of claims 1 to 8 is used for recognition reliability in respect of exhaust gases of diesel engines, catalytic converter engines or conventional Otto engines.

## Revendications

1. Agencement de détecteur de gaz, avec au moins un premier élément détecteur (11) qui est conformé en élément détecteur à d'oxyde stannique, est essentiellement sensible aux gaz oxydables présents dans l'air extérieur et produit un signal de mesure correspondant, avec au moins un deuxième élément détecteur (21) et une unité électrique de contrôle et de traitement, à laquelle sont envoyés les signaux de mesure produits par les premier et deuxième éléments détecteurs (11, 21), **caractérisé en ce que** le deuxième élément détecteur (21) est essentiellement sensible aux gaz réductibles dans l'air extérieur et produit un signal de mesure correspondant, et **en ce que** le signal de mesure du premier élément détecteur (11) et le signal de mesure du deuxième élément détecteur (21) sont disponibles au niveau d'un point de prélèvement (13) commun, le premier élément détecteur (11) et le deuxième élément détecteur (21) formant un diviseur de tension qui comprend le point de prélèvement (13) et l'agencement fournissant un signal de commutation aussi bien en présence de gaz d'échappement de moteurs Diesel qu'en présence de gaz d'échappement de moteurs à allumage commandé.

2. Agencement de détecteur de gaz selon la revendication 1, dans lequel le deuxième élément détecteur (21) est un élément détecteur à conduction de type N à base de semi-conducteur homogène, et au niveau de la couche sensible au gaz, est formé d'oxyde métallique.

3. Agencement de détecteur de gaz selon la revendication 2, dans lequel le deuxième élément détecteur (21), au niveau de la couche sensible au gaz, est formé d'une couche d'oxyde stannique réduit.

4. Agencement de détecteur de gaz selon la revendication 2 ou 3, dans lequel le deuxième élément détecteur (21) présente des additifs catalytiques et une fine couche de métal précieux est appliquée sur la couche sensible au gaz.

5. Agencement de détecteur de gaz selon une des revendications 2 à 4, dans lequel le deuxième élément détecteur (21) est entouré d'un fil chauffé en un matériau catalytique, tel que du platine, de l'or, du rhodium ou du palladium.

6. Agencement de détecteur de gaz selon la revendication 2, dans lequel le deuxième élément détecteur (21) est formé d'oxyde stannique avec des additifs catalytiques, d'oxyde de zinc réduit avec des additifs catalytiques ou d'oxyde de fer avec des additifs catalytiques.

7. Agencement de détecteur de gaz selon la revendication 2, dans lequel le deuxième élément détecteur (21) est formé d'un mélange d'au moins deux des matériaux cités dans la revendication 6.

8. Agencement de détecteur de gaz selon une des revendication 2 à 7, dans lequel on utilise comme additifs catalytiques dans le deuxième élément détecteur (21) du palladium, du platine, du rhodium ou de l'or.

9. Procédé de détection de gaz, dans lequel pour la de détection fiable des gaz d'échappement de moteurs Diesel, de moteurs à catalyseur ou de moteurs à allumage commandé traditionnels on utilise un agencement de détecteur de gaz selon une des revendications 1 à 8.
